(19) Europäisches Patentamt
European Patent Office
Office européen des brevets

(11) EP 3 356 320 B1

(12) FASCICULE DE BREVET EUROPEEN

(45) Date de publication et mention de la délivrance du brevet:
**03.07.2019 Bulletin 2019/27**

(21) Numéro de dépôt: **16775801.0**

(22) Date de dépôt: **07.09.2016**

(51) Int Cl.:
***C07C 51/42*** (2006.01) ***C07C 69/67*** (2006.01)

(86) Numéro de dépôt international:
**PCT/FR2016/052221**

(87) Numéro de publication internationale:
**WO 2017/055698 (06.04.2017 Gazette 2017/14)**

# (54) PROCEDE DE SYNTHESE D'OLIGOMERES D'ACIDE ACRYLIQUE

VERFAHREN ZUR SYNTHESE VON ACRYLSÄUREOLIGOMEREN

METHOD FOR THE SYNTHESIS OF OLIGOMERS OF ACRYLIC ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.10.2015 FR 1559390**

(43) Date de publication de la demande:
**08.08.2018 Bulletin 2018/32**

(73) Titulaire: **Arkema France**
**92700 Colombes (FR)**

(72) Inventeurs:
- **LINEMANN, Reinhard**
  **D-66121 Saarbrucken (DE)**
- **RIFLADE, Benoit**
  **33430 Bazas (FR)**
- **TRETJAK, Serge**
  **57520 Roulhing (FR)**
- **LEVRAY, André**
  **57890 Porcelette (FR)**
- **DEFER, Patrice**
  **57530 Courcelles-chaussy (FR)**

(74) Mandataire: **Bonnel, Claudine**
**ARKEMA France**
**Département Propriété Industrielle**
**420, rue d'Estienne d'Orves**
**92705 Colombes Cedex (FR)**

(56) Documents cités:
**US-A- 4 359 564**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 356 320 B1

Printed by Jouve, 75001 PARIS (FR)

## Description

### DOMAINE TECHNIQUE

La présente invention concerne la synthèse d'un monomère acrylique, en particulier d'un oligomère d'acide acrylique, représenté par la formule (I) :

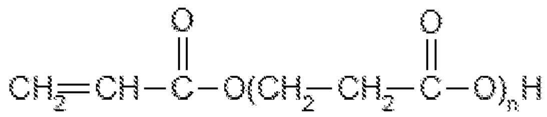

dans laquelle n est un entier allant de 1 à 10,
utilisable comme (co)monomère pour fabriquer des polymères acryliques.

### ART ANTERIEUR

Il est connu du brevet US 4,267,365 de préparer des oligomères d'acide acrylique répondant à la formule (I) dans laquelle n est compris entre 1 et 6, de préférence entre 1 et 3, par chauffage d'acide acrylique à une température allant d'environ 50°C à 200°C en présence d'un inhibiteur de polymérisation à une teneur allant de 0,001 à 1% en poids par rapport à la quantité d'acide acrylique. Le procédé est mis en oeuvre sous une pression comprise généralement entre 20 Torr et 50 atmosphères, éventuellement en présence d'un solvant inerte. En adaptant la température et/ou le temps de séjour, il est possible d'obtenir majoritairement le dimère d'acide acrylique ou majoritairement des oligomères de chaine plus longue. Le mélange obtenu selon ce procédé contient en poids de 1 à 99% d'acide acrylique non polymérisé, qui peut être distillé pour être recyclé dans le procédé après ajout d'un inhibiteur de polymérisation. Alternativement, le mélange est utilisé tel quel sans purification pour la préparation de dérivés d'acide polyacrylique dans le domaine des adhésifs.

Le brevet US 4,359,564 décrit la synthèse d'oligomères d'acide acrylique utilisés en tant que co-monomères dans des polymérisations en solution ou en émulsion. Selon un mode de réalisation, la synthèse est réalisée à partir d'acide acrylique en présence d'un éther couronne et d'une faible teneur en acrylate de potassium, et en présence d'éther méthylique d'hydroquinone (EMHQ, inhibiteur de polymérisation), à une température de 80°C pendant 300 heures. Le degré moyen d'oligomérisation de l'acide acrylique est de l'ordre de 3. Selon un autre mode de réalisation, l'acide acrylique est chauffé à une température plus élevée, de 120-125°C, pendant une durée plus courte, au contact d'une résine échangeuse d'ions fortement acide en présence d'un mélange de deux inhibiteurs de polymérisation. Selon la durée de réaction, le degré moyen d'oligomérisation de l'acide acrylique est de 1,4 ou 2.

Selon les procédés de l'art antérieur, la synthèse d'oligomères d'acide acrylique s'effectue toujours en présence d'un inhibiteur de polymérisation à une teneur relativement élevée, et conduit à un produit final fortement stabilisé, ce qui peut être préjudiciable pour l'utilisation de ce produit comme co-monomère dans des procédés de polymérisation.

En outre, en fonction des applications envisagées des (co)polymères obtenus à partir d'oligomères d'acide acrylique, il peut être avantageux de disposer d'oligomères d'acide acrylique présentant une distribution contrôlée des espèces oligomériques.

Il subsiste donc un besoin de produire des oligomères d'acide acrylique en forte concentration, comportant à la fois une faible teneur en stabilisant, et une distribution contrôlée des espèces oligomériques.

De façon surprenante, il a été trouvé que la présence d'une faible quantité d'eau en association avec un catalyseur acide ou basique permet de réduire la consommation d'inhibiteur de polymérisation au cours de la réaction tout en contrôlant la concentration et la distribution des espèces oligomériques lors du chauffage de l'acide acrylique à une température modérée.

Cette diminution de la consommation en inhibiteur de polymérisation permet de baisser et de contrôler globalement la teneur en inhibiteur au cours de la production d'oligomères et d'obtenir une teneur finale d'inhibiteur dans le produit plus faible qui est avantageuse pour l'utilisation de ces oligomères dans des procédés de polymérisation.

### RESUME DE L'INVENTION

L'invention a donc pour objet un procédé de synthèse d'un oligomère d'acide acrylique représenté par la formule (I) :

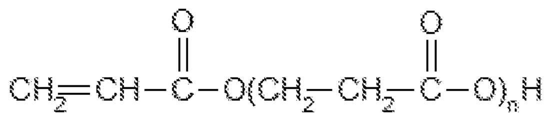

dans laquelle n est un entier allant de 1 à 10, de préférence de 1 à 6 caractérisé en ce qu'il comprend le chauffage d'acide acrylique à une température allant de 50°C à 200°C en présence d'un catalyseur, d'eau, et d'au moins un inhibiteur de polymérisation.

La formule (I) dans laquelle n=0 représente l'acide acrylique, précurseur pour préparer l'oligomère d'acide acrylique.

Selon un mode de réalisation, n est un entier allant de 1 à 10, de préférence de 1 à 6, par exemple de 1 à 4.

Dans la suite du texte, les expressions « compris entre ... et ... », « allant de ... à ... » et « variant de ... à ... » sont équivalentes et entendent signifier que les bornes sont incluses, sauf mention contraire.

Les expressions « espèces oligomères », « oligomères » ou « espèces oligomériques » sont équivalentes, et excluent les polymères d'acide acrylique de

degré de polymérisation supérieur à 10.

Par « oligomérisation », on entend une polymérisation par addition de Michael conduisant à des espèces oligomériques.

Selon l'invention, l'oligomère d'acide acrylique se trouve sous la forme d'un liquide à température ambiante comprenant un mélange d'espèces acides de formule (I) de différentes longueurs de chaine selon la valeur de n, telles que le dimère d'acide acrylique (dénommé aussi acide 3-(acryloyloxy)propionique, n=1), le trimère d'acide acrylique (n=2), le tétramère d'acide acrylique (n=3), etc. Le liquide peut contenir de l'acide acrylique non réagi (n=0), et de l'eau. Il est au moins partiellement soluble dans l'eau.

Pour simplification, dans la suite de l'exposé, on dénommera par di-AA le dimère d'acide acrylique, par tri-AA le trimère d'acide acrylique et par oligo-AA les espèces oligomères pour lesquels n > 2 dans la formule (I).

Le procédé selon l'invention peut comprendre en outre une étape de purification comprenant au moins une distillation pour éliminer l'eau et/ou l'acide acrylique résiduel.

L'oligomère d'acide acrylique représenté par la formule (I) susceptible d'être obtenu selon le procédé selon l'invention, comprend de 10 à 2000 ppm d'inhibiteur de polymérisation, de préférence de 50 à 2000 ppm, en particulier de 100 à 1000 ppm, encore plus particulièrement de 200 à 500 ppm d'inhibiteur de polymérisation.

L'oligomère d'acide acrylique de formule (I) susceptible d'être obtenu selon le procédé selon l'invention, est utilisé comme (co)monomère pour préparer des polymères acryliques.

Un autre objet de l'invention est un procédé de synthèse d'un oligomère d'acide acrylique représenté par la formule (I) :

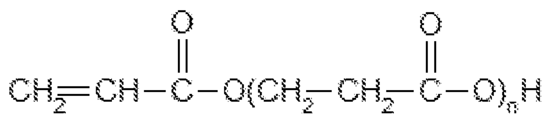

dans laquelle n est un entier allant de 1 à 10, de préférence de 1 à 6, caractérisé en ce qu'il comprend les étapes suivantes :

a) On chauffe de l'acide acrylique à une température allant de 50°C à 200°C dans un réacteur comprenant un catalyseur, en présence d'eau, et en présence d'au moins un inhibiteur de polymérisation, conduisant à un mélange réactionnel comprenant des oligomères d'acide acrylique, de l'acide acrylique, de l'eau et des inhibiteurs de polymérisation ;

b) On soumet le mélange réactionnel à une première distillation permettant de séparer un flux de tête comprenant de l'eau et de l'acide acrylique, qui peut être au moins partiellement renvoyé dans le réacteur, et en pied un flux comprenant des oligomères d'acide acrylique, de l'acide acrylique résiduel et des inhibiteurs de polymérisation ;

c) On soumet le flux de pied à une seconde une distillation permettant de séparer un flux comprenant essentiellement de l'acide acrylique et un flux comprenant essentiellement l'oligomère d'acide acrylique de formule (I) et les inhibiteurs de polymérisation ;

d) On recycle le flux d'acide acrylique à l'étape a) ;

e) De façon optionnelle, on soumet le flux comprenant l'oligomère d'acide acrylique obtenu à l'étape c) à un évaporateur à film permettant de séparer, d'une part un flux constitué essentiellement de dimères et trimères d'acide acrylique et d'autre part un flux constitué essentiellement d'oligomères d'acide acrylique répondant à la formule (I) dans laquelle n est un entier allant de 2 à 10, de préférence allant de 3 à 10.

La distillation effectuée à l'étape b) et à l'étape c) peut être réalisée à l'aide une colonne de distillation classique ou à l'aide d'un évaporateur à film tombant.

On peut également soumettre le flux issu de l'étape b) à d'autres procédés de séparation telle que la cristallisation ou l'extraction liquide-liquide.

La présente invention permet de surmonter les inconvénients de l'état de la technique. Elle fournit plus particulièrement un procédé de synthèse d'un oligomère d'acide acrylique permettant d'ajuster la composition des espèces oligomères présentes, et comportant un faible taux de stabilisant (autre dénomination pour inhibiteur de polymérisation).

Ceci est accompli grâce à la présence d'eau qui permet de stabiliser le mélange réactionnel en présence d'un catalyseur qui peut être acide ou basique, et de réduire la quantité d'inhibiteur de polymérisation à mettre en oeuvre pour maitriser l'oligomérisation de l'acide acrylique. La consommation d'inhibiteur de polymérisation étant réduite grâce à la combinaison des conditions opératoires mises en oeuvre pour la réaction thermique de l'acide acrylique, il est possible d'ajuster la quantité d'inhibiteur de polymérisation à introduire dans le milieu réactionnel à la quantité souhaitée dans l'oligomère produit.

## BREVE DESCRIPTION DES FIGURES

La Figure 1 représente de manière schématique un mode de réalisation du procédé selon l'invention.

## DESCRIPTION DETAILLEE DE L'INVENTION

L'invention est maintenant décrite plus en détail et de façon non limitative dans la description qui suit.

Dans le procédé de l'invention, on utilise de l'acide acrylique comme matière première qui est soumise à un traitement thermique sous certaines conditions de façon à provoquer une oligomérisation contrôlée dudit

acide conduisant à un mélange d'espèces oligomères de longueur variable.

L'acide acrylique peut être d'origine pétrochimique ou au moins en partie d'origine renouvelable.

Selon un mode de réalisation de l'invention, l'acide acrylique est d'origine pétrochimique et dérive d'un procédé de production utilisant le propylène ou le propane comme matière première.

Selon un mode de réalisation de l'invention, l'acide acrylique dérive d'un procédé utilisant l'acide acétique comme matière première.

Selon un mode de réalisation de l'invention, l'acide acrylique est d'origine renouvelable et dérive d'un procédé de production utilisant le glycérol ou la glycérine comme matière première.

Selon un mode de réalisation de l'invention, l'acide acrylique est obtenu à partir de sucres.

Selon un mode de réalisation, l'acide acrylique est d'origine renouvelable et il est issu d'un procédé de déshydratation de l'acide lactique ou du lactate d'ammonium en acide acrylique, ou d'un procédé de déshydratation de l'acide 3-hydroxypropionique ou de son sel d'ammonium en acide acrylique.

Le procédé selon l'invention comprend une réaction thermique à une température allant de 50°C à 200°C, de préférence de 80°C à 140°C, par exemple comprise entre 90°C et 125°C.

On opère généralement sous pression normale ou sous pression réduite, la pression allant de la pression atmosphérique à environ 50 mbars, ou en surpression, la pression pouvant aller jusqu'à 20 bars.

L'acide acrylique est mis en contact avec un catalyseur. Le catalyseur peut être acide ou basique. Le catalyseur peut être homogène ou hétérogène.

Comme catalyseur acide homogène, on peut utiliser par exemple un acide organique sulfonique, comme l'acide méthane sulfonique, l'acide para-toluène sulfonique, l'acide benzène sulfonique, l'acide dodécylsulfonique, l'acide xylène sulfonique, ou leurs mélanges, ou l'acide sulfurique, ou les composés acryliques polymérisables tel que l'acide sulfonique 2-acrylamide-2-méthyl propane (AMPS) commercialisé par la société Lubrizol.

Comme catalyseur acide hétérogène, on peut utiliser par exemple une résine échangeuse d'ions, par exemple une résine cationique sulfonée, ou une zéolithe fortement acide. Le catalyseur est avantageusement une résine cationique forte de type styrène/divinylbenzène comportant des groupements sulfoniques. A titre d'exemples de résines, on peut citer la résine Amberlyst A16 ou la résine Amberlyst A15 commercialisées par la société Dow.

Comme catalyseur basique homogène, on peut utiliser par exemple la soude, la potasse, des carbonates ou des bases aminées.

Comme catalyseur basique hétérogène, on peut utiliser par exemple une résine échangeuse d'ions fortement basique, par exemple une résine anionique. A titre d'exemples de résines, on peut citer la résine Amberlyst A28 commercialisée par la société Dow.

Comme catalyseurs utilisables, on peut citer encore des catalyseurs métalliques ou acide de Lewis tels que le chlorure de fer, le chlorure d'aluminium, l'acétate de palladium, l'hydroxyde de palladium.

La quantité massique de catalyseur homogène est généralement comprise entre 0,1 et 20%, de préférence entre 1 et 10 % par rapport à la masse d'acide acrylique.

On conduit la réaction thermique en présence d'au moins un inhibiteur de polymérisation choisi par exemple parmi la phénothiazine, l'hydroquinone, l'éther monométhylique d'hydroquinone (EMHQ), les phénols encombrés tel que le ditertbutyl para-crésol (BHT) ou le di-tertiobutylcatéchol, la paraphénylène diamine, le TEMPO (2,2,6,6-tétraméthyl-1-piperidinyloxy), ou les dérivés du TEMPO, tel que le OH-TEMPO, seuls ou leurs mélanges en toutes proportions. De préférence, on utilise l'EMHQ comme inhibiteur de polymérisation.

Le milieu réactionnel contient une faible quantité d'au moins un inhibiteur de polymérisation par rapport au poids total d'acide acrylique mis en oeuvre dans le procédé. La quantité massique d'inhibiteur de polymérisation mise en oeuvre est comprise entre 10 et 2000 ppm, de préférence entre 50 et 2000 ppm, en particulier entre 100 et 1000 ppm, encore plus particulièrement entre 200 et 500 ppm par rapport à la masse d'acide acrylique.

Selon un mode de réalisation intéressant de la présente invention, on utilise une association d'un inhibiteur de polymérisation de type phénolique, par exemple l'hydroquinone ou l'éther monométhylique d'hydroquinone, à une teneur allant de 10 à 2000 ppm, de préférence de 100 à 1000 ppm avec un inhibiteur de polymérisation azoté de type TEMPO ou un dérivé de TEMPO à une teneur allant de 1 à 200 ppm, de préférence de 10 à 60 ppm.

La réaction thermique d'oligomerisation d'acide acrylique se fait avec une injection contrôlée d'air appauvri en oxygène quand les inhibiteurs de polymérisation sont des composés phénoliques.

Selon l'invention, le milieu réactionnel contient une faible quantité d'eau par rapport au poids total d'acide acrylique mis en oeuvre dans le procédé. La quantité d'eau mise en oeuvre pour préparer l'oligomère d'acide acrylique peut représenter en masse de 0,01 à 20 %, de préférence de 0,1 à 5 %, en particulier de 0,5 à 3% par rapport à la masse d'acide acrylique.

Selon un mode de réalisation de la présente invention, on utilise un catalyseur basique tel que la soude ou la potasse, et l'eau présente dans le milieu réactionnel provient de la réaction de l'acide acrylique avec le catalyseur, il n'est alors pas nécessaire d'ajouter de l'eau au milieu réactionnel.

Le temps de réaction thermique est généralement compris entre 1 et 20 heures.

La réaction thermique peut être effectuée dans un réacteur agité, ou dans plusieurs réacteurs agités en

cascades, équipés d'une double enveloppe, ou dans un réacteur en boucle brassée contenant un lit fixe de catalyseur, des dispositifs de sécurité étant associés aux réacteurs (disque de rupture, injection d'un agent d'arrêt de polymérisation en cas d'emballement de la réaction, mise en refroidissement de la masse réactionnelle, contrôle de la pression).

Le procédé selon l'invention peut être mis en oeuvre en batch, en semi continu avec une introduction progressive de l'acide acrylique, ou en continu.

L'introduction des inhibiteurs de polymérisation peut être effectuée de manière séparée, ou par utilisation d'un acide acrylique préalablement stabilisé avec la quantité d'inhibiteur désirée.

Le procédé selon l'invention permet d'obtenir un oligomère d'acide acrylique de formule (I) comprenant une teneur finale en inhibiteur de polymérisation allant de 10 à 2000 ppm, de préférence de 50 à 2000 ppm, en particulier de 100 à 1000 ppm, encore plus particulièrement de 200 à 500 ppm, tout à fait compatible avec son utilisation comme (co)monomère dans des procédés de polymérisation par voie radicalaire.

Le procédé selon l'invention permet d'obtenir un oligomère d'acide acrylique de formule (I), généralement en mélange avec de l'acide acrylique libre (à l'état non polymérisé) dans des proportions massiques pouvant aller de 1/99 à 99/1, de préférence de 20/80 à 99/1 (exprimé en proportion massique oligomère/acide).

Selon un mode de réalisation, la teneur en acide acrylique résiduel est inférieure à 60% en masse, par exemple inférieure à 40% ou à 20% ou à 10% en masse par rapport à la masse totale de l'oligomère.

Le procédé selon l'invention permet d'obtenir un oligomère d'acide acrylique comprenant une teneur élevée en dimères di-AA et trimères tri-AA, par exemple supérieure à 20% en masse, en particulier supérieure à 22% ou à 24% en masse par rapport à la masse totale de l'oligomère.

L'oligomère d'acide acrylique obtenu selon le procédé selon l'invention peut être caractérisé par un degré moyen d'oligomérisation.

On définit le degré moyen d'oligomérisation par une moyenne en nombre des espèces d'oligomères obtenues selon le procédé de l'invention. Il peut notamment être déterminé à partir d'une mesure de l'indice d'acide du liquide obtenu. L'Indice d'Acide (IA en méq d'acide par gramme) est déterminé grâce à un dosage potentiométrique.

Le nombre moyen N d'oligomérisation est déterminé grâce à l'indice d'acide IA selon la formule :

$$N = -1{,}443 \ln (IA) + 3{,}7946$$

Le degré moyen d'oligomérisation de l'oligomère d'acide acrylique obtenu selon le procédé de l'invention est compris entre 0,1 et 10, de préférence entre 0,1 et 3 pour un produit essentiellement constitué d'une fraction riche en dimère et trimères, ou entre 3 à 10 pour un produit qui contient essentiellement des oligomères de formule (I) avec n>2.

Le procédé selon l'invention permet en outre de limiter la formation d'impuretés dans l'oligomère d'acide acrylique, par exemple la formation d'acide 3-hydroxypropionique (3-HPA). La teneur en 3-HPA est généralement inférieure à 1% massique, par exemple inférieure à 0,8% massique par rapport à l'oligomère formé dans les conditions du procédé selon l'invention.

La réaction d'oligomérisation de l'acide acrylique peut être contrôlée en réalisant un suivi de l'acidité qui diminue au cours de la réaction thermique.

Selon un mode de réalisation, le procédé selon l'invention comprend en outre une étape de purification comprenant au moins une distillation pour éliminer l'eau et/ou l'acide acrylique résiduel. Avantageusement, l'étape de purification comprend une première distillation permettant de séparer l'eau et l'acide acrylique, et un évaporateur à film pour séparer les dimères et trimères des oligomères de masse moléculaire plus élevée.

Avantageusement, l'acide acrylique ainsi que l'eau sont renvoyés à l'étape de réaction thermique.

En référence à la Figure 1, qui représente un mode de réalisation préféré du procédé selon l'invention, on alimente un réacteur R avec un flux 1 d'acide acrylique et un flux 2 d'eau. L'acide acrylique contient la quantité désirée d'inhibiteur de polymérisation. Le réacteur comprend un lit fixe d'une résine échangeuse d'ions, maintenu à une température allant de 50°C à 200°C et de préférence à la pression atmosphérique. Les débits d'alimentation sont réglés de façon à avoir un temps de séjour adapté à la conversion souhaitée pour l'acide acrylique.

Le mélange réactionnel 3 en sortie du réacteur est soumis à une distillation dans une colonne C séparant, en tête un flux 6 comprenant l'eau et de l'acide acrylique, et en pied un flux 5 comprenant les oligomères d'acide acrylique formés dans le réacteur R, l'acide acrylique qui n'est pas transformé en oligomère et la quantité d'inhibiteur de polymérisation qui n'a pas été consommée lors du traitement thermique.

Le flux de tête 6 est avantageusement recyclé en partie à la réaction via le flux 10, l'autre partie 11 étant envoyée vers une station de traitement des eaux.

Le flux 5 comprenant l'oligomère d'acide acrylique de formule (I) est soumis à une colonne de distillation C1 pour séparer l'acide acrylique résiduel qui est avantageusement recyclé dans le réacteur via le flux 4.

Un traitement final à l'aide d'un évaporateur à film E peut être effectué sur le flux 7 d'oligomères qui a été débarrassé de l'acide acrylique résiduel afin d'isoler un mélange 9 constitué principalement de dimère et trimère d'acide acrylique, de l'ensemble des oligomères présents.

Selon un mode de réalisation, l'oligomère d'acide acrylique obtenu selon le procédé de l'invention est utilisé tel quel sans étape de séparation de l'acide acrylique résiduel, comme (co)monomère pour fabriquer des

polymères acryliques.

Selon un mode de réalisation, on élimine l'acide acrylique résiduel avant d'utiliser l'oligomère d'acide acrylique obtenu selon le procédé de l'invention comme (co)monomère pour fabriquer des polymères acryliques.

Selon un mode de réalisation, on utilise le mélange de dimère et trimère d'acide acrylique obtenu selon le procédé de l'invention comme (co)monomère pour fabriquer des polymères acryliques.

Les polymères acryliques comprenant au moins un monomère constitué d'un oligomère d'acide acrylique obtenu selon l'invention sont avantageusement utilisés pour préparer des dispersants ou des épaississants acryliques, des adhésifs hydrophiles ou des revêtements acryliques.

L'invention est maintenant illustrée par les exemples suivants, qui n'ont pas pour but de limiter la portée de l'invention, définie par les revendications annexées.

**EXEMPLES**

Sauf indication contraire, les pourcentages sont exprimés en pourcentages massiques.

Les abréviations suivantes sont utilisées :

AA : Acide acrylique
di-AA : dimère d'acide acrylique
tri-AA : trimère d'acide acrylique
oligo-AA :

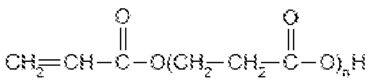

avec n >2
EMHQ : ester méthylique d'hydroquinone
4-OH-T : 4-hydroxy-TEMPO
A16 : Résine Amberlyst A16

Les teneurs en AA, di-AA, tri-AA et EMHQ dans les différents essais de synthèse ont été déterminées par analyse HPLC. Les teneurs mesurées d'EMHQ par HPLC ont une marge d'erreur de l'ordre de 2%.

La détermination des teneurs en AA, di-AA et tri-AA permet d'estimer la teneur en oligo-AA (par différence à 100).

L'Indice d'Acide (IA en méq d'acide par gramme) est déterminé grâce à un dosage potentiometrique. Plus l'indice d'acide est faible, plus grande sera la concentration en oligomère.

Le nombre moyen N d'oligomérisation sera déterminé grâce à l'indice d'acide IA selon la formule :

$$N = -1{,}443\ln(IA) + 3{,}7946$$

**Exemple 1 (comparatif)** : essai en batch en l'absence de résine et d'eau

Dans un réacteur tricol, parfaitement agité, équipé d'une sonde de température et surmonté d'un réfrigérant, on a introduit :

200 g d'AA
0% d'eau
0% de résine A16
1023 ppm d'EMHQ

Le milieu réactionnel a été agité pendant 8 heures à 97°C sous bullage d'air.

Après 8h, le milieu réactionnel comprend :

- AA : 92,5%
- di-AA : 7,06%
- tri-AA : 0,2%
- Teneur en EMHQ finale : 1022 ppm. Consommation d'EMHQ : 0%

IA = 13,46 meq/g
N = 0,043

La teneur en diAA est inférieure à 10%.

Dans cet essai, la consommation d'EMHQ est nulle.

**Exemple 2 (comparatif)** : essai en batch en présence de résine et en l'absence d'eau

L'exemple 1 a été repris en présence de résine A16 :

200 g d'AA
0% d'eau
20% de résine A16
1264 ppm d'EMHQ

Après 8h, le milieu réactionnel comprend :

- AA : 83,55 %
- di-AA : 13,71%
- tri-AA : 1,31%
- Teneur en EMHQ finale : 524 ppm. Consommation d'EMHQ : 58.5%
- IA = 12,49 meq/g
- N = 0,151

La présence de la résine permet d'augmenter de manière importante le rendement en dimère et trimère d'acide acrylique qui a doublé par rapport à l'exemple 1. Le nombre moyen d'oligomérisation a triplé par rapport à l'exemple 1. Cependant la résine engendre une consommation très importante en stabilisant EMHQ (>50%).

**Exemple 3 (selon l'invention)** : essai en batch en présence de résine et d'eau

L'exemple 1 a été repris avec l'ajout d'eau et en présence de résine A16 :

200 g d'AA
1% d'eau
20% de résine A16
661 ppm d'EMHQ

Après 8h, le milieu réactionnel comprend :

- AA : 80.06 %
- di-AA : 12,89%
- tri-AA : 1,47%
- Teneur en EMHQ finale : 581 ppm. Consommation d'EMHQ : 12%
- IA = 12,21 meq/g
- N = 0,184

Le rendement total en dimère et trimère est similaire à l'exemple 2. Mais l'ajout d'1% d'eau a permis de limiter de manière très importante la consommation en EMHQ (12% vs 58,5%).

**Exemple 4 (selon invention)** : essai en batch en présence de résine et d'eau, effet de la température.

L'exemple 3 a été repris à 107°C :

200 g d'AA
1% d'eau
20% de résine A16
642 ppm d'EMHQ

Après 8h, le milieu réactionnel comprend :

- AA: 59.41%
- di-AA : 22,41%
- tri-AA : 4,94%
- Teneur en EMHQ finale : 545 ppm. Consommation d'EMHQ : 15%
- IA = 10,69 meq/g
- N = 0,376

L'augmentation de la température de 10°C a permis d'augmenter le rendement en dimère + trimère de manière très importante. L'indice d'acidité a baissé et le nombre moyen d'oligomérisation a augmenté. La présence d'1% d'eau a permis de limiter de nouveau la consommation d'EMHQ

**Exemple 5 (comparatif) : selon le brevet US 4,359,564**

Dans un réacteur tricol, parfaitement agité, équipé d'une sonde de température et surmonté d'un réfrigérant, on a introduit :

100 g d'AA
0% d'eau
20% de résine A15
1342 ppm d'EMHQ

Le milieu réactionnel a été agité pendant 9,5 heures à 125°C sous bullage d'air.

Après 9,5 h, le milieu réactionnel comprend :

- AA : 24,06%
- di-AA : 19,22%
- tri-AA : 10%
- Teneur en EMHQ finale : 532 ppm. Consommation d'EMHQ : 60%
- IA = 7,35 meq/g
- N = 0,915

La consommation en AA est importante, le nombre moyen d'oligomérisation est égal à 0,915 démontrant la présence d'espèces oligomériques, et le taux de dimères important. Cependant la consommation en EMHQ est toujours très importante (60%).

**Exemple 6 (selon l'invention)**

L'exemple 5 a été repris avec :

100 g d'AA
5% d'eau
20% de résine A15
1484 ppm d'EMHQ

Le milieu réactionnel a été agité pendant 9.5 heures à 125°C sous bullage d'air.

Après 9.5 h, le milieu réactionnel comprend :

- AA : 22,04%
- di-AA : 17,3%
- tri-AA : 8,51%
- Teneur en EMHQ finale : 1048 ppm. Consommation d'EMHQ : 30%
- IA = 7,08 meq/g
- N=0,971

Les résultats en indice d'oligomérisation, di-AA et tri-AA sont similaires. L'ajout d'eau a été bénéfique pour la consommation en stabilisant EMHQ.

**Exemple 7 (selon l'invention)** : essai en présence d'un catalyseur basique et en présence d'eau

Dans un réacteur tricol, parfaitement agité, équipé d'une sonde de température et surmonté d'un réfrigérant, on a introduit :

100 g d'AA
1% d'eau
1% de soude

589 ppm d'EMHQ

Le milieu réactionnel a été agité pendant 8 heures à 97°C sous bullage d'air.
Après 8 h, le milieu réactionnel comprend :

- AA : 65,24%
- di-AA : 22,46%
- tri-AA : 2,98%
- Teneur en EMHQ finale : 594 ppm. Consommation d'EMHQ : 0%
- IA = 10,96 meq/g
- N = 0,339

Par rapport aux exemples 1, 2 et 3 à la même température, les teneurs en dimères et trimères sont plus importantes, la catalyse basique permet d'améliorer la réaction d'oligomérisation de l'acide acrylique. La présence d'eau permet également d'éviter toute consommation en EMHQ.

<u>**Exemple 8 (selon l'invention)**</u> : essai en continu

Un mélange réactionnel constitué d'AA, d'eau et d'EMHQ alimente en continu un réacteur en verre d'un volume de 160 mL à l'aide une pompe à membrane. Ce réacteur en verre est constitué d'un préchauffeur, relié à une colonne en verre contenant un lit fixe de catalyseur (Résine A16), chauffé à l'aide d'un bain d'huile double enveloppe, surmonté d'un réfrigérant et relié à un condenseur. L'ensemble est calorifugé. Un bullage d'air en continu est assuré.
L'alimentation contient : AA, 12000 ppm d'eau, et 961 ppm d'EMHQ. Le débit d'alimentation est de 60 mL/h
Après un temps de séjour de 2,6 heures à 107°C, la composition du milieu est la suivante :

- di-AA : 22,17%
- tri-AA : 5,46%
- Teneur en EMHQ finale : 699 ppm. Consommation d'EMHQ : 27.2%
- IA = 10,48 meq/g
- N = 0,405

Sur un lit de résine fixe, à la même température que l'exemple 3, le nombre moyen d'oligomérisation est supérieur alors que le temps de séjour a été divisé par 4. La présence de 12000 ppm d'eau permet de limiter la consommation en EMHQ.

<u>**Exemple 9 (selon l'invention)**</u> : essai en continu

L'exemple 8 a été repris avec l'alimentation suivante : AA, 6000 ppm d'eau, 723 ppm d'EMHQ
Après un temps de séjour de 2,4 h à 102°C, la composition du milieu est la suivante :

- di-AA : 23,12%
- tri-AA : 6,23%
- Teneur en EMHQ finale : 469 ppm. Consommation d'EMHQ : 35%
- IA = 10,23 meq/g
- N = 0,439

La diminution de la teneur en eau au départ par rapport à l'exemple 8, accélère la consommation d'EMHQ. Cela prouve une fois de plus l'intérêt bénéfique de l'eau avec la résine pour limiter la consommation d'EMHQ.

**Revendications**

1. Procédé de synthèse d'un oligomère d'acide acrylique représenté par la formule (I) :

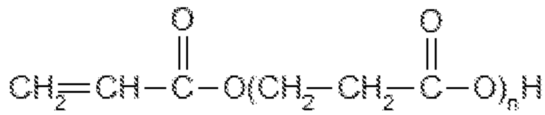

dans laquelle n est un entier allant de 1 à 10, de préférence de 1 à 6, **caractérisé en ce qu'**il comprend le chauffage d'acide acrylique à une température allant de 50°C à 200°C en présence d'un catalyseur, d'eau, et d'au moins un inhibiteur de polymérisation.

2. Procédé selon la revendication 1 **caractérisé en ce que** l'acide acrylique est d'origine pétrochimique.

3. Procédé selon la revendication 1 **caractérisé en ce que** l'acide acrylique est au moins en partie d'origine renouvelable.

4. Procédé selon l'une quelconque des revendications précédentes **caractérisé en ce que** le catalyseur est un catalyseur acide homogène tel qu'un acide organique sulfonique, ou l'acide sulfurique, ou un catalyseur acide hétérogène tel qu'une résine échangeuse d'ions ou une zéolithe fortement acide

5. Procédé selon l'une quelconque des revendications 1 à 3 **caractérisé en ce que** le catalyseur est un catalyseur basique homogène tel que la soude, la potasse, des carbonates ou des bases aminées, ou un catalyseur basique hétérogène tel qu'une résine échangeuse d'ions fortement basique.

6. Procédé selon la revendication 5 **caractérisé en ce que** le catalyseur basique est la soude ou la potasse, et l'eau présente dans le milieu réactionnel provient de la réaction de l'acide acrylique avec le catalyseur, sans ajout d'eau dans le milieu réactionnel.

7. Procédé selon l'une quelconque des revendications précédentes **caractérisé en ce que** la quantité massique d'eau mise en oeuvre représente de 0,01% à 20% par rapport à la masse d'acide acrylique, de préférence de 0,1% à 5%, en particulier de 0,5 à 3%.

8. Procédé selon l'une quelconque des revendications précédentes **caractérisé en ce que** la quantité massique d'inhibiteur de polymérisation mise en oeuvre est comprise entre 10 et 2000 ppm, de préférence entre 50 et 2000 ppm, en particulier entre 100 et 1000 ppm, par rapport à la masse d'acide acrylique.

9. Procédé selon l'une quelconque des revendications précédentes **caractérisé en ce que** l'inhibiteur de polymérisation est choisi parmi la phénothiazine, l'hydroquinone, l'éther monométhylique d'hydroquinone, les phénols encombrés tel que le diterbutyl para-crésol (BHT) ou le di-tertiobutylcatéchol, la paraphénylène diamine, le TEMPO (2,2,6,6-tétraméthyl-1-piperidinyloxy), ou les dérivés du TEMPO, tel que le OH-TEMPO, seuls ou leurs mélanges en toutes proportions.

10. Procédé selon l'une quelconque des revendications précédentes **caractérisé en ce qu'**il comprend en outre une étape de purification comprenant au moins une distillation pour éliminer l'eau et/ou l'acide acrylique résiduel.

11. Procédé de synthèse d'un oligomère d'acide acrylique représenté par la formule (I) :

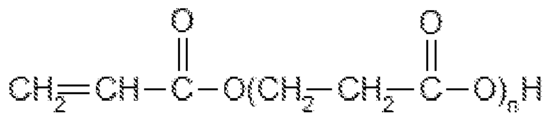

dans laquelle n est un entier allant de 1 à 10, de préférence de 1 à 6, **caractérisé en ce qu'**il comprend les étapes suivantes :

a) On chauffe de l'acide acrylique à une température allant de 50°C à 200°C dans un réacteur comprenant un catalyseur, en présence d'eau, et en présence d'au moins un inhibiteur de polymérisation, conduisant à un mélange réactionnel comprenant des oligomères d'acide acrylique, de l'acide acrylique, de l'eau et des inhibiteurs de polymérisation ;
b) On soumet le mélange réactionnel à une première distillation permettant de séparer un flux de tête comprenant de l'eau et de l'acide acrylique, qui peut être au moins partiellement renvoyé dans le réacteur, et en pied un flux comprenant des oligomères d'acide acrylique, de l'acide acrylique résiduel et des inhibiteurs de polymérisation ;
c) On soumet le flux de pied à une seconde distillation permettant de séparer un flux comprenant essentiellement de l'acide acrylique et un flux comprenant essentiellement l'oligomère d'acide acrylique de formule (I) et les inhibiteurs de polymérisation ;
d) On recycle le flux d'acide acrylique à l'étape a) ;
e) De façon optionnelle, on soumet le flux comprenant l'oligomère d'acide acrylique obtenu à l'étape c) à un évaporateur à film permettant de séparer, d'une part un flux constitué essentiellement de dimères et trimères d'acide acrylique et d'autre part un flux constitué essentiellement d'oligomères d'acide acrylique répondant à la formule (I) dans laquelle n est un entier allant de 2 à 10, de préférence allant de 3 à 10.

**Patentansprüche**

1. Verfahren zur Synthese eines Acrylsäure-Oligomers der Formel (I):

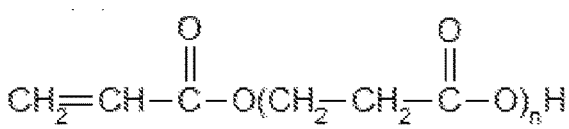

worin n für eine ganze Zahl im Bereich von 1 bis 10, vorzugsweise von 1 bis 6, steht,
**dadurch gekennzeichnet, dass** es das Erhitzen von Acrylsäure auf eine Temperatur im Bereich von 50 °C bis 200 °C in Gegenwart von einem Katalysator, Wasser und mindestens einem Polymerisationsinhibitor umfasst.

2. Verfahren nach Anspruch 1, **dadurch gekennzeichnet, dass** die Acrylsäure petrochemischen Ursprungs ist.

3. Verfahren nach Anspruch 1, **dadurch gekennzeichnet, dass** die Acrylsäure zumindest teilweise erneuerbaren Ursprungs ist.

4. Verfahren nach einem der vorhergehenden Ansprüche, **dadurch gekennzeichnet, dass** es sich bei dem Katalysator um einen homogenen sauren Katalysator wie eine organische Sulfonsäure oder Schwefelsäure oder einen heterogenen sauren Katalysator wie ein Ionenaustauscherharz oder einen stark sauren Zeolith handelt.

5. Verfahren nach einem der Ansprüche 1 bis 3, **dadurch gekennzeichnet, dass** es sich bei dem Katalysator um einen homogenen basischen Katalysator wie Natriumhydroxid, Kaliumhydroxid, Carbonate oder Amin-Basen oder einen heterogenen basischen Katalysator wie ein stark basisches Ionenaustauscherharz handelt.

6. Verfahren nach Anspruch 5, **dadurch gekennzeichnet, dass** es sich bei dem basischen Katalysator um Natriumhydroxid oder Kaliumhydroxid handelt und das im Reaktionsmedium vorliegende Wasser aus der Reaktion der Acrylsäure mit dem Katalysator stammt, ohne dass Wasser in das Reaktionsmedium gegeben wird.

7. Verfahren nach einem der vorhergehenden Ansprüche, **dadurch gekennzeichnet, dass** die eingesetzte Gewichtsmenge von Wasser 0,01 bis 20 %, bezogen auf das Gewicht der Acrylsäure, vorzugsweise 0,1 bis 5 %, insbesondere 0,5 bis 3 %, ausmacht.

8. Verfahren nach einem der vorhergehenden Ansprüche, **dadurch gekennzeichnet, dass** die eingesetzte Gewichtsmenge von Polymerisationsinhibitor zwischen 10 und 2000 ppm, vorzugsweise zwischen 50 und 2000 ppm, insbesondere zwischen 100 und 1000 ppm, bezogen auf das Gewicht der Acrylsäure, liegt.

9. Verfahren nach einem der vorhergehenden Ansprüche, **dadurch gekennzeichnet, dass** der Polymerisationsinhibitor aus Phenothiazin, Hydrochinon, Hydrochinonmonomethylether, gehinderten Phenolen wie Di-tert-butyl-para-kresol (BHT) oder Di-tert-butylcatechol, para-Phenylendiamin, TEMPO (2,2,6,6-Tetramethyl-1-piperidinyloxy) oder TEMPO-Derivaten, wie OH-TEMPO, allein oder Mischungen davon in allen Verhältnissen ausgewählt wird.

10. Verfahren nach einem der vorhergehenden Ansprüche, **dadurch gekennzeichnet, dass** es außerdem einen Reinigungsschritt umfasst, der mindestens eine Destillation zur Entfernung des Wassers und/oder der restlichen Acrylsäure umfasst.

11. Verfahren zur Synthese eines Acrylsäure-Oligomers der Formel (I):

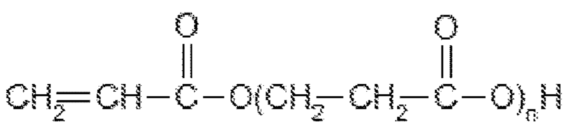

worin n für eine ganze Zahl im Bereich von 1 bis 10, vorzugsweise von 1 bis 6, steht,
**dadurch gekennzeichnet, dass** es die folgenden Schritte umfasst:

a) Acrylsäure wird in einem Reaktor, der einen Katalysator umfasst, in Gegenwart von Wasser und in Gegenwart von mindestens einem Polymerisationsinhibitor auf eine Temperatur im Bereich von 50 °C bis 200 °C erhitzt, was eine Reaktionsmischung ergibt, die Acrylsäure-Oligomere, Acrylsäure, Wasser und Polymerisationsinhibitoren umfasst;
b) die Reaktionsmischung wird einer ersten Destillation unterworfen, die die Abtrennung eines Kopfstroms, der Wasser und Acrylsäure umfasst und zumindest teilweise in den Reaktor zurückgeführt werden kann, und eines Sumpfstroms, der Acrylsäure-Oligomere, restliche Acrylsäure und Polymerisationsinhibitoren umfasst, ermöglicht;
c) der Sumpfstrom wird einer zweiten Destillation unterworfen, die die Abtrennung eines Stroms, der im Wesentlichen Acrylsäure umfasst, und eines Stroms, der im Wesentlichen Acrylsäure-Oligomer der Formel (I) und Polymerisationsinhibitoren umfasst, ermöglicht;
d) der Acrylsäurestrom wird zu Schritt a) zurückgeführt;
e) gegebenenfalls wird der in Schritt c) erhaltene Strom, der Acrylsäure-Oligomer umfasst, einem Filmverdampfer unterworfen, der die Abtrennung eines Stroms, der im Wesentlichen aus Acrylsäure-Dimeren und -Trimeren besteht, einerseits und eines Stroms, der im Wesentlichen aus Acrylsäure-Oligomeren der Formel (I), worin n für eine ganze Zahl im Bereich von 2 bis 10, vorzugsweise im Bereich von 3 bis 10, steht, besteht, andererseits ermöglicht.

## Claims

1. Process for the synthesis of an acrylic acid oligomer represented by the formula (I):

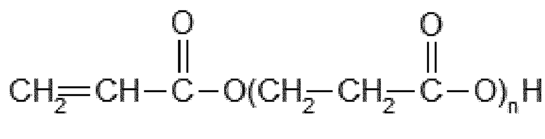

in which n is an integer ranging from 1 to 10, preferably from 1 to 6, **characterized in that** it comprises heating acrylic acid at a temperature ranging from 50°C to 200°C in the presence of a catalyst, of water and of at least one polymerization inhibitor.

2. Process according to Claim 1, **characterized in that** the acrylic acid is of petrochemical origin.

3. Process according to Claim 1, **characterized in that** the acrylic acid is at least partly of renewable origin.

**4.** Process according to any one of the preceding claims, **characterized in that** the catalyst is a homogeneous acid catalyst such as a sulphonic organic acid, or sulphuric acid, or a heterogeneous acid catalyst such as an ion exchange resin or a strongly acidic zeolite.

**5.** Process according to any one of Claims 1 to 3, **characterized in that** the catalyst is a homogeneous basic catalyst such as sodium hydroxide, potassium hydroxide, carbonates or amino bases, or a heterogeneous basic catalyst such as a strongly basic ion exchange resin.

**6.** Process according to Claim 5, **characterized in that** the basic catalyst is sodium hydroxide or potassium hydroxide, and the water present in the reaction medium comes from the reaction of the acrylic acid with the catalyst, without the addition of water to the reaction medium.

**7.** Process according to any one of the preceding claims, **characterized in that** the weight amount of water used represents from 0.01% to 20% relative to the weight of acrylic acid, preferably from 0.1% to 5%, in particular from 0.5% to 3%.

**8.** Process according to any one of the preceding claims, **characterized in that** the weight amount of polymerization inhibitor used is between 10 and 2000 ppm, preferably between 50 and 2000 ppm, in particular between 100 and 1000 ppm, relative to the weight of acrylic acid.

**9.** Process according to any one of the preceding claims, **characterized in that** the polymerization inhibitor is chosen from phenothiazine, hydroquinone, hydroquinone monomethyl ether, hindered phenols such as di-tert-butyl para-cresol (BHT) or di-tert-butylcatechol, paraphenylenediamine, TEMPO (2,2,6,6-tetramethyl-1-piperidinyloxy), or TEMPO derivatives, such as OH-TEMPO, alone or mixtures thereof in any proportions.

**10.** Process according to any one of the preceding claims, **characterized in that** it also comprises a purification step comprising at least one distillation in order to eliminate the water and/or the residual acrylic acid.

**11.** Process for the synthesis of an acrylic acid oligomer represented by formula (I):

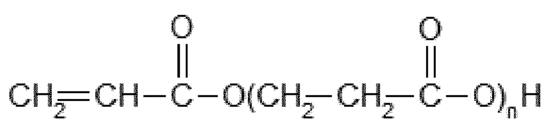

in which n is an integer ranging from 1 to 10, preferably from 1 to 6, **characterized in that** it comprises the following steps:

a) acrylic acid is heated at a temperature ranging from 50°C to 200°C in a reactor comprising a catalyst, in the presence of water, and in the presence of at least one polymerization inhibitor, resulting in a reaction mixture comprising acrylic acid oligomers, acrylic acid, water and polymerization inhibitors;
b) the reaction mixture is subjected to a first distillation making it possible to separate a top stream comprising water and acrylic acid, which can be at least partially sent back to the reactor, and at the bottom a stream comprising acrylic acid oligomers, residual acrylic acid and polymerization inhibitors;
c) the bottom stream is subjected to a second distillation making it possible to separate a stream comprising essentially acrylic acid and a stream comprising essentially the acrylic acid oligomer of formula (I) and the polymerization inhibitors;
d) the acrylic acid stream is recycled to step a);
e) optionally, the stream comprising the acrylic acid oligomer obtained in step c) is subjected to a film evaporator which makes it possible to separate, on the one hand, a stream consisting essentially of acrylic acid dimers and trimers and, on the other hand, a stream consisting essentially of acrylic acid oligomers, corresponding to formula (I) in which n is an integer ranging from 2 to 10, preferably from 3 to 10.

FIGURE 1

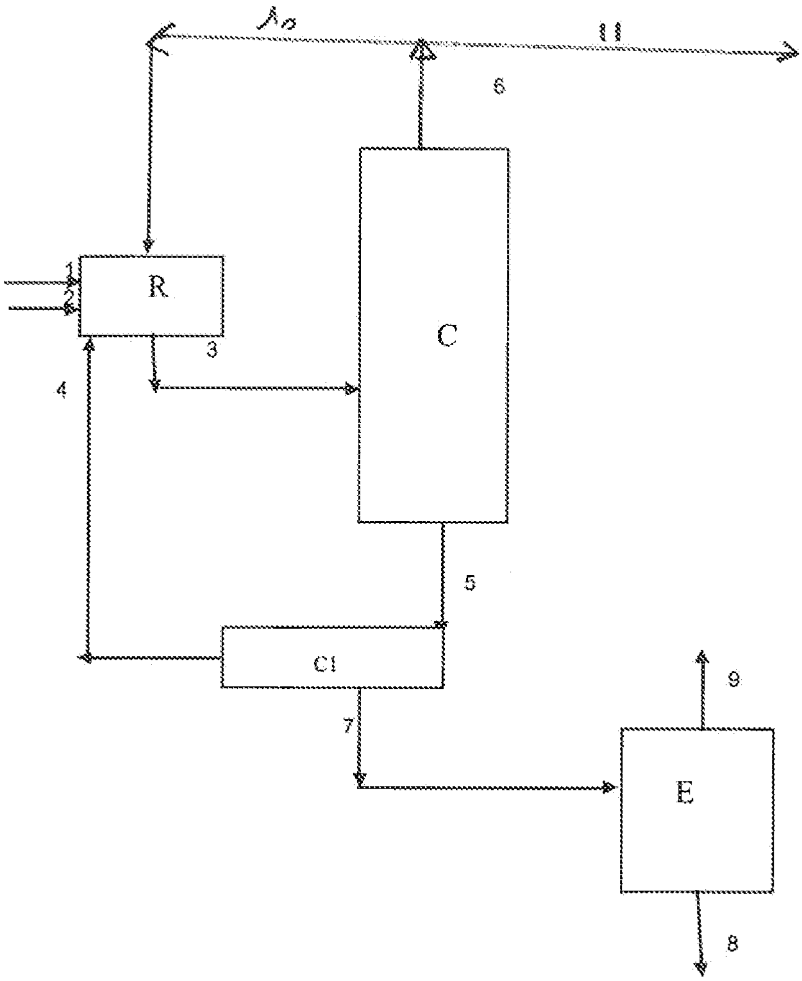

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4267365 A
- US 4359564 A